# EUROPEAN PATENT SPECIFICATION

(11) **EP 3 082 855 B1**
(45) Date of publication and mention of the grant of the patent: **24.02.2021**
(21) Application number: 14828499.5
(22) Date of filing: 18.12.2014
(51) Int. Cl.: A61K 39/08, C07K 14/245, C07K 14/33, A61K 39/00

(54) **COMPOSITION**
ZUSAMMENSETZUNG
COMPOSITION

(30) Priority: 18.12.2013 GB 201322463
(43) Date of publication of application: 26.10.2016
(73) Proprietor: University of Exeter, Exeter EX4 4QJ (GB); Universiteit Gent, 9000 Gent (BE)
(72) Inventor: TITBALL, Richard William, Devon EX4 4QJ (GB); FERNANDES DA COSTA, Sergio Paulo, 65933 Frankfurt am Main (DE); VAN IMMERSEEL, Filip, 9810 Eke (BE); DUCATELLE, Richard, 9790 Wortegem-Petegem (BE); MOT, Dorien, 9506 Waarbeke (BE)
(74) Representative: Greaves Brewster LLP
(86) International application number: PCT/GB2014/053748
(87) International publication number: WO 2015/092406

(56) References cited:
- WO-A1-93/23543
- WO-A1-2013/061056
- FERNANDES DA COSTA SÉRGIO P ET AL: "Protection against avian necrotic enteritis after immunisation with NetB genetic or formaldehyde toxoids", VACCINE, vol. 31, no. 37, 20 May 2013 (2013-05-20), pages 4003-4008, XP028690341, ISSN: 0264-410X, DOI: 10.1016/J.VACCINE.2013.05.063

### SEQUENCE LISTING

<110> University of Exeter Ghent University
<120> Composition
<130> DS/P2299PC00
<150> GB1322463.9
   <151> 2013-12-18
<160> 21
<170> PatentIn version 3.5
<210> 1
   <211> 292
   <212> PRT
   <213> Clostridium perfringens
<400> 1
   Ser Glu Leu Asn Asp Ile Asn Lys Ile Glu Leu Lys Asn Leu Ser Gly
   1               5                   10                  15
   Glu Ile Ile Lys Glu Asn Gly Lys Glu Ala Ile Lys Tyr Thr Ser Ser
               20                  25                  30
   Asp Thr Ala Ser His Lys Gly Trp Lys Ala Thr Leu Ser Gly Thr Phe
           35                  40                  45
   Ile Glu Asp Pro His Ser Asp Lys Lys Thr Ala Leu Leu Asn Leu Glu
       50                  55                  60
   Gly Phe Ile Pro Ser Asp Lys Gln Ile Phe Gly Ser Lys Tyr Tyr Gly
   65                  70                  75                  80
   Lys Met Lys Trp Pro Glu Thr Tyr Arg Ile Asn Val Lys Ser Ala Asp
                   85                  90                  95
   Val Asn Asn Asn Ile Lys Ile Ala Asn Ser Ile Pro Lys Asn Thr Ile
               100                 105                 110
   Asp Lys Lys Asp Val Ser Asn Ser Ile Gly Tyr Ser Ile Gly Gly Asn
           115                 120                 125
   Ile Ser Val Glu Gly Lys Thr Ala Gly Ala Gly Ile Asn Ala Ser Tyr
       130                 135                 140
   Asn Val Gln Asn Thr Ile Ser Tyr Glu Gln Pro Asp Phe Arg Thr Ile
   145                 150                 155                 160
   Gln Arg Lys Asp Asp Ala Asn Leu Ala Ser Trp Asp Ile Lys Phe Val
                   165                 170                 175
   Glu Thr Lys Asp Gly Tyr Asn Ile Asp Ser Tyr His Ala Ile Tyr Gly
               180                 185                 190
   Asn Gln Leu Phe Met Lys Ser Arg Leu Tyr Asn Asn Gly Asp Lys Asn
           195                 200                 205
   Phe Thr Asp Asp Arg Asp Leu Ser Thr Leu Ile Ser Gly Gly Phe Ser
       210                 215                 220
   Pro Asn Met Ala Leu Ala Leu Thr Ala Pro Lys Asn Ala Lys Glu Ser
   225                 230                 235                 240
   Val Ile Ile Val Glu Tyr Gln Arg Phe Asp Asn Asp Tyr Ile Leu Asn
                   245                 250                 255
   Trp Glu Thr Thr Gln Trp Arg Gly Thr Asn Lys Leu Ser Ser Thr Ser
               260                 265                 270
   Glu Tyr Asn Glu Phe Met Phe Lys Ile Asn Trp Gln Asp His Lys Ile
           275                 280                 285
   Glu Tyr Tyr Leu
       290
<210> 2
   <211> 292
   <212> PRT
   <213> Artificial Sequence
<220>
   <223> Substituted sequence
<400> 2
   Ser Glu Leu Asn Asp Ile Asn Lys Ile Glu Leu Lys Asn Leu Ser Gly
   1               5                   10                  15
   Glu Ile Ile Lys Glu Asn Gly Lys Glu Ala Ile Lys Tyr Thr Ser Ser
               20                  25                  30
   Asp Thr Ala Ser His Lys Gly Trp Lys Ala Thr Leu Ser Gly Thr Phe
           35                  40                  45
   Ile Glu Asp Pro His Ser Asp Lys Lys Thr Ala Leu Leu Asn Leu Glu
       50                  55                  60
   Gly Phe Ile Pro Ser Asp Lys Gln Ile Phe Gly Ser Lys Tyr Tyr Gly
   65 70 75 80
   Lys Met Lys Trp Pro Glu Thr Tyr Arg Ile Asn Val Lys Ser Ala Asp
   85 90 95
   Val Asn Asn Asn Ile Lys Ile Ala Asn Ser Ile Pro Lys Asn Thr Ile
   100 105 110
   Asp Lys Lys Asp Val Ser Asn Ser Ile Gly Tyr Ser Ile Gly Gly Asn
   115 120 125
   Ile Ser Val Glu Gly Lys Thr Ala Gly Ala Gly Ile Asn Ala Ser Tyr
   130 135 140
   Asn Val Gln Asn Thr Ile Ser Tyr Glu Gln Pro Asp Phe Arg Thr Ile
   145 150 155 160
   Gln Arg Lys Asp Asp Ala Asn Leu Ala Ser Trp Asp Ile Lys Phe Val
   165 170 175
   Glu Thr Lys Asp Gly Tyr Asn Ile Asp Ser Tyr His Ala Ile Tyr Gly
   180 185 190
   Asn Gln Leu Phe Met Lys Ser Arg Leu Tyr Asn Asn Gly Asp Lys Asn
   195 200 205
   Phe Thr Asp Asp Arg Asp Leu Ser Thr Leu Ile Ser Gly Gly Phe Ser
   210 215 220
   Pro Asn Met Ala Leu Ala Leu Thr Ala Pro Lys Asn Ala Lys Glu Ser
   225 230 235 240
   Val Ile Ile Val Glu Tyr Gln Arg Phe Asp Asn Asp Tyr Ile Leu Asn
   245 250 255
   Trp Glu Thr Thr Gln Ala Arg Gly Thr Asn Lys Leu Ser Ser Thr Ser
   260 265 270
   Glu Tyr Asn Glu Phe Met Phe Lys Ile Asn Trp Gln Asp His Lys Ile
   275 280 285
   Glu Tyr Tyr Leu
   290
<210> 3
   <211> 124
   <212> PRT
   <213> Clostridium perfringens
<400> 3
   Asn Asp Pro Ser Val Gly Lys Asn Val Lys Glu Leu Val Ala Tyr Ile
   1               5                   10                  15
   Ser Thr Ser Gly Glu Lys Asp Ala Gly Thr Asp Asp Tyr Met Tyr Phe
               20                  25                  30
   Gly Ile Lys Thr Lys Asp Gly Lys Thr Gln Glu Trp Glu Met Asp Asn
           35                  40                  45
   Pro Gly Asn Asp Phe Met Thr Gly Ser Lys Asp Thr Tyr Thr Phe Lys
       50                  55                  60
   Leu Lys Asp Glu Asn Leu Lys Ile Asp Asp Ile Gln Asn Met Trp Ile
   65                  70                  75                  80
   Arg Lys Arg Lys Tyr Thr Ala Phe Ser Asp Ala Tyr Lys Pro Glu Asn
                   85                  90                  95
   Ile Lys Ile Ile Ala Asn Gly Lys Val Val Val Asp Lys Asp Ile Asn
               100                 105                 110
   Glu Trp Ile Ser Gly Asn Ser Thr Tyr Asn Ile Lys
           115                 120
<210> 4
   <211> 10
   <212> **PRT**
   <213> **Artificial** Sequence
<220>
   <223> Mutant peptide fragment
<220>
   <221> Xaa
   <222> (5)..(5)
   <223> Any amino acid other than Y
<400> 4
   Tyr His Ala Ile Xaa Gly Asn Gln Leu Phe
   1               5                   10
<210> 5
   <211> 20
   <212> PRT
   <213> Artificial Sequence
<220>
   <223> Mutant peptide fragment
<220>
   <221> Xaa
   <222> (10)..(10)
   <223> Any amino acid other than Y
<400> 5
   Tyr Asn Ile Asp Ser Tyr His Ala Ile Xaa Gly Asn Gln Leu Phe Met
   1               5                   10                  15
   Lys Ser Arg Leu
               20
<210> 6
   <211> 10
   <212> PRT
   <213> Artificial Sequence
<220>
   <223> Mutant peptide fragment
<220>
   <221> Xaa
   <222> (5)..(5)
   <223> Any amino acid other than R
<400> 6
   Phe Met Lys Ser Xaa Leu Tyr Asn Asn Gly
   1               5                   10
<210> 7
   <211> 20
   <212> PRT
   <213> Artificial Sequence
<220>
   <223> Mutant peptide fragment
<220>
   <221> Xaa
   <222> (10)..(10)
   <223> Any amino acid other than R
<400> 7
   Tyr Gly Asn Gln Leu Phe Met Lys Ser Xaa Leu Tyr Asn Asn Gly Asp
   1               5                   10                  15
   Lys Asn Phe Thr
               20
<210> 8
   <211> 10
   <212> PRT
   <213> Artificial Sequence
<220>
   <223> Mutant peptide fragment
<220>
   <221> Xaa
   <222> (5)..(5)
   <223> Any amino acid other than W
<400> 8
   Tyr Ile Leu Asn Xaa Glu Thr Thr Gln Trp
   1               5                   10
<210> 9
   <211> 20
   <212> PRT
   <213> Artificial Sequence
<220>
   <223> Mutant peptide fragment
<220>
   <221> Xaa
   <222> (10)..(10)
   <223> Any amino acid other than W
<400> 9
   Arg Phe Asp Asn Asp Tyr Ile Leu Asn Xaa Glu Thr Thr Gln Trp Arg
   1               5                   10                  15
   Gly Thr Asn Lys
               20
<210> 10
   <211> 10
   <212> PRT
   <213> Artificial Sequence
<220>
   <223> Mutant peptide fragment
<220>
   <221> Xaa
   <222> (5)..(5)
   <223> Any amino acid other than W
<400> 10
   Glu Thr Thr Gln Xaa Arg Gly Thr Asn Lys
   1               5                   10
<210> 11
   <211> 20
   <212> PRT
   <213> Artificial Sequence
<220>
   <223> Mutant peptide fragment
<220>
   <221> Xaa
   <222> (10)..(10)
   <223> Any amino acid other than W
<400> 11
   Tyr Ile Leu Asn Trp Glu Thr Thr Gln Xaa Arg Gly Thr Asn Lys Leu
   1 5 10 15
   Ser Ser Thr Ser
   20
<210> 12
   <211> 5
   <212> **PRT**
   <213> **Artificial** Sequence
<220>
   <223> Protein fragment
<400> 12
   Thr Asp Asp Tyr Met
   1 5
<210> 13
   <211> 10
   <212> PRT
   <213> Artificial Sequence
<220>
   <223> Protein fragment
<400> 13
   Asp Ala Gly Thr Asp Asp Tyr Met Tyr Phe
   1 5 10
<210> 14
   <211> 5
   <212> PRT
   <213> Artificial Sequence
<220>
   <223> Protein fragment
<400> 14
   Asn Pro Gly Asn Asp
   1 5
<210> 15
   <211> 10
   <212> PRT
   <213> Artificial Sequence
<220>
   <223> Protein fragment
<400> 15
   Glu Met Asp Asn Pro Gly Asn Asp Phe Met
   1               5                   10
<210> 16
   <211> 32
   <212> PRT
   <213> Artificial Sequence
<220>
   <223> Protein fragment
<400> 16
   Asp Ala Gly Thr Asp Asp Tyr Met Tyr Phe Gly Ile Lys Thr Lys Asp
   1               5                   10                  15
   Gly Lys Thr Gln Glu Trp Glu Met Asp Asn Pro Gly Asn Asp Phe Met
               20                  25                  30
<210> 17
   <211> 10
   <212> PRT
   <213> Artificial Sequence
<220>
   <223> Mutant peptide fragment
<400> 17
   Glu Thr Thr Gln Ala Arg Gly Thr Asn Lys
   1               5                   10
<210> 18
   <211> 370
   <212> PRT
   <213> Clostridium perfringens
<400> 18
   Trp Asp Gly Lys Ile Asp Gly Thr Gly Thr His Ala Met Ile Val Thr
   1               5                   10                  15
   Gln Gly Val Ser Ile Leu Glu Asn Asp Leu Ser Lys Asn Glu Pro Glu
               20                  25                  30
   Ser Val Arg Lys Asn Leu Glu Ile Leu Lys Glu Asn Met His Glu Leu
           35                  40                  45
   Gln Leu Gly Ser Thr Tyr Pro Asp Tyr Asp Lys Asn Ala Tyr Asp Leu
       50                  55                  60
   Tyr Gln Asp His Phe Trp Asp Pro Asp Thr Asp Asn Asn Phe Ser Lys
   65                  70                  75                  80
   Asp Asn Ser Trp Tyr Leu Ala Tyr Ser Ile Pro Asp Thr Gly Glu Ser
                   85                  90                  95
   Gln Ile Arg Lys Phe Ser Ala Leu Ala Arg Tyr Glu Trp Gln Arg Gly
               100                 105                 110
   Asn Tyr Lys Gln Ala Thr Phe Tyr Leu Gly Glu Ala Met His Tyr Phe
           115                 120                 125
   Gly Asp Ile Asp Thr Pro Tyr His Pro Ala Asn Val Thr Ala Val Asp
       130                 135                 140
   Ser Ala Gly His Val Lys Phe Glu Thr Phe Ala Glu Glu Arg Lys Glu
   145                 150                 155                 160
   Gln Tyr Lys Ile Asn Thr Ala Gly Cys Lys Thr Asn Glu Ala Phe Tyr
                   165                 170                 175
   Thr Asp Ile Leu Lys Asn Lys Asp Phe Asn Ala Trp Ser Lys Glu Tyr
               180                 185                 190
   Ala Arg Gly Phe Ala Lys Thr Gly Lys Ser Ile Tyr Tyr Ser His Ala
           195                 200                 205
   Ser Met Ser His Ser Trp Asp Asp Trp Asp Tyr Ala Ala Lys Val Thr
       210                 215                 220
   Leu Ala Asn Ser Gln Lys Gly Thr Ala Gly Tyr Ile Tyr Arg Phe Leu
   225                 230                 235                 240
   His Asp Val Ser Glu Gly Asn Asp Pro Ser Val Gly Lys Asn Val Lys
                   245                 250                 255
   Glu Leu Val Ala Tyr Ile Ser Thr Ser Gly Glu Lys Asp Ala Gly Thr
               260                 265                 270
   Asp Asp Tyr Met Tyr Phe Gly Ile Lys Thr Lys Asp Gly Lys Thr Gln
           275                 280                 285
   Glu Trp Glu Met Asp Asn Pro Gly Asn Asp Phe Met Thr Gly Ser Lys
       290                 295                 300
   Asp Thr Tyr Thr Phe Lys Leu Lys Asp Glu Asn Leu Lys Ile Asp Asp
   305                 310                 315                 320
   Ile Gln Asn Met Trp Ile Arg Lys Arg Lys Tyr Thr Ala Phe Ser Asp
                   325                 330                 335
   Ala Tyr Lys Pro Glu Asn Ile Lys Ile Ile Ala Asn Gly Lys Val Val
               340                 345                 350
   Val Asp Lys Asp Ile Asn Glu Trp Ile Ser Gly Asn Ser Thr Tyr Asn
           355                 360                 365
   Ile Lys
       370
<210> 19
   <211> 331
   <212> PRT
   <213> Artificial Sequence
<220>
   <223> Expression construct
<400> 19
   Met Gly Gly Ser His His His His His His Gly Met Ala Ser Met Thr
   1               5                   10                  15
   Gly Gly Gln Gln Met Gly Arg Asp Leu Tyr Asp Asp Asp Asp Lys Asp
               20                  25                  30
   Arg Trp Gly Ser Glu Leu Glu Ser Glu Leu Asn Asp Ile Asn Lys Ile
           35                  40                  45
   Glu Leu Lys Asn Leu Ser Gly Glu Ile Ile Lys Glu Asn Gly Lys Glu
       50                  55                  60
   Ala Ile Lys Tyr Thr Ser Ser Asp Thr Ala Ser His Lys Gly Trp Lys
   65                  70                  75                  80
   Ala Thr Leu Ser Gly Thr Phe Ile Glu Asp Pro His Ser Asp Lys Lys
                   85                  90                  95
   Thr Ala Leu Leu Asn Leu Glu Gly Phe Ile Pro Ser Asp Lys Gln Ile
               100                 105                 110
   Phe Gly Ser Lys Tyr Tyr Gly Lys Met Lys Trp Pro Glu Thr Tyr Arg
                   115                 120                 125
   Ile Asn Val Lys Ser Ala Asp Val Asn Asn Asn Ile Lys Ile Ala Asn
       130                 135                 140
   Ser Ile Pro Lys Asn Thr Ile Asp Lys Lys Asp Val Ser Asn Ser Ile
   145                 150                 155                 160
   Gly Tyr Ser Ile Gly Gly Asn Ile Ser Val Glu Gly Lys Thr Ala Gly
                   165                 170                 175
   Ala Gly Ile Asn Ala Ser Tyr Asn Val Gln Asn Thr Ile Ser Tyr Glu
               180                 185                 190
   Gln Pro Asp Phe Arg Thr Ile Gln Arg Lys Asp Asp Ala Asn Leu Ala
           195                 200                 205
   Ser Trp Asp Ile Lys Phe Val Glu Thr Lys Asp Gly Tyr Asn Ile Asp
       210                 215                 220
   Ser Tyr His Ala Ile Tyr Gly Asn Gln Leu Phe Met Lys Ser Arg Leu
   225                 230                 235                 240
   Tyr Asn Asn Gly Asp Lys Asn Phe Thr Asp Asp Arg Asp Leu Ser Thr
                   245                 250                 255
   Leu Ile Ser Gly Gly Phe Ser Pro Asn Met Ala Leu Ala Leu Thr Ala
               260                 265                 270
   Pro Lys Asn Ala Lys Glu Ser Val Ile Ile Val Glu Tyr Gln Arg Phe
           275                 280                 285
   Asp Asn Asp Tyr Ile Leu Asn Trp Glu Thr Thr Gln Ala Arg Gly Thr
       290                 295                 300
   Asn Lys Leu Ser Ser Thr Ser Glu Tyr Asn Glu Phe Met Phe Lys Ile
   305                 310                 315                 320
   Asn Trp Gln Asp His Lys Ile Glu Tyr Tyr Leu
                   325                 330
<210> 20
   <211> 353
   <212> PRT
   <213> Artificial Sequence
<220>
   <223> Expression construct
<400> 20
   Met Ser Pro Ile Leu Gly Tyr Trp Lys Ile Lys Gly Leu Val Gln Pro
   1               5                   10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145                 150                 155                 160
   Val Val Leu Tyr Met Asp Pro Met Cys Leu Asp Ala Phe Pro Lys Leu
                   165                 170                 175
   Val Cys Phe Lys Lys Arg Ile Glu Ala Ile Pro Gln Ile Asp Lys Tyr
               180                 185                 190
   Leu Lys Ser Ser Lys Tyr Ile Ala Trp Pro Leu Gln Gly Trp Gln Ala
           195                 200                 205
   Thr Phe Gly Gly Gly Asp His Pro Pro Lys Ser Asp Leu Ile Glu Gly
       210                 215                 220
   Arg Gly Ile Pro Gly Asn Asp Pro Ser Val Gly Lys Asn Val Lys Glu
   225                 230                 235                 240
   Leu Val Ala Tyr Ile Ser Thr Ser Gly Glu Lys Asp Ala Gly Thr Asp
   245 250 255
   Asp Tyr Met Tyr Phe Gly Ile Lys Thr Lys Asp Gly Lys Thr Gln Glu
   260 265 270
   Trp Glu Met Asp Asn Pro Gly Asn Asp Phe Met Thr Gly Ser Lys Asp
   275 280 285
   Thr Tyr Thr Phe Lys Leu Lys Asp Glu Asn Leu Lys Ile Asp Asp Ile
   290 295 300
   Gln Asn Met Trp Ile Arg Lys Arg Lys Tyr Thr Ala Phe Ser Asp Ala
   305 310 315 320
   Tyr Lys Pro Glu Asn Ile Lys Ile Ile Ala Asn Gly Lys Val Val Val
   325 330 335
   Asp Lys Asp Ile Asn Glu Trp Ile Ser Gly Asn Ser Thr Tyr Asn Ile
   340 345 350
   Lys
<210> 21
   <211> 6
   <212> PRT
   <213> Artificial Sequence
<220>
   <223> Linker
<400> 21
   Gly Ser Gly Gly Gly Gly
   1 5

## Description

### Field of the Invention

The invention relates to a composition comprising two or more polypeptides which, when administered together, provide complete protection to a subject against infection by *Clostridium perfringens.*

### Background

*Clostridium perfringens* (*C. perfringens*) is a ubiquitous bacterium that is able to colonise a variety of different biotopes and it is not unusual to find *C*. *perfringens* as a commensal in the normal gut microbiota in domesticated animals. However, under particular circumstances the bacterium is responsible for severe diseases. These diseases are largely a consequence of the actions of toxins on the host [1]. In addition to the four toxins used for typing *C*. *perfringens* strains (alpha-, beta-, epsilon-, and iota-toxin), the bacterium is able to produce a number of other toxins, including enterotoxin and perfringolysin O [2, 3].

*C. perfringens* has been shown to cause avian necrotic enteritis (NE), a severe gastro-intestinal disease of farmed poultry [4-6]. Until recently, NE has been controlled by the addition of antimicrobial growth promoters to feedstuffs. However, in many countries national and supranational regulations now limit the addition of antimicrobials to animal feeds. Consequently, in these countries, NE is emerging as a disease which is of significant economic consequence to the poultry industry [7, 8]. The disease can occur in at least two forms. The acute form of NE typically results in mortality during the last weeks of rear of broilers (week 5-6). However, many cases of NE are associated with relatively mild clinical signs [9-11]. This subclinical form of NE results in decreased digestion and absorption of feedstuffs and consequently reduced weight gain [12, 13]. At least in Europe it is now believed that the subclinical NE is the most frequent form of the disease and causes the greatest economic losses to the poultry production industry [14].

Although it is clear that *C. perfringens* is the etiologic agent of NE, a wide range of host and pathogen factors can influence the severity of the disease. These factors include the nature of the feedstuff, co-infection with various *Eimeria* species and the molecular makeup of *C*. *perfringens* in the gut [15]. Often these factors interact with each other, and this has made the development of reliable infection models difficult [15]. The molecular basis of virulence of *C**.** perfringens* associated with NE is still being investigated. However, almost all *C*. *perfringens* isolates from cases of NE possess the *netB* gene [4, 16, 17] which encodes necrotic enteritis toxin B (NetB), a β-pore-forming toxin [6, 18]. Pore formation by NetB can lead to cell lysis by disruption of membrane integrity and a *netB* mutant of *C*. *perfringens* is reported to be incapable of causing NE [6]. There is also accumulating evidence that other virulence factors, such as the TpeL toxin, play a role in disease [19]. The production of these virulence factors might explain the reported ability of some *netB*-negative strains of *C*. *perfringens* to cause NE [4, 6].

Immunisation with either crude toxoids [20] or culture supernatants [21] can provide significant but incomplete protection against experimental NE. Although these vaccines are simple to prepare they suffer from the limitation that it is difficult to configure them for non-invasive dosing, for example by oral delivery. Other workers have explored the possibility of a sub-unit which is able to protect against NE, with a view towards both improving vaccine efficacy and opening the possibility of oral delivery. To date, a range of proteins derived from *C. perfringens* have been evaluated as sub-unit vaccines including alpha-toxin, glyceraldehyde-3-phosphate dehydrogenase, pyruvate-ferredoxin oxidoreductase, fructose 1,6-biphosphate-aldolase, or a hypothetical protein [22]. Immunisation with any of these subunits provided partial protection against experimental NE. Partial protection against NE has also been reported after immunisation with *C*. *perfringens* large cytotoxin TpeL, endo-beta-N-acetylglucosaminidase or phosphoglyceromutase [23]. A more recent study in which alpha-toxin, NetB, pyruvate-ferredoxin oxidoreductase and elongation factor-Tu were compared as protective antigens concluded that NetB and pyruvate-ferredoxin oxidoreductase given with ISA71 adjuvant provided enhanced protective immunity [24]. However, it is unlikely that a licensed vaccine for widespread use could contain active toxins. Therefore, there is a need to identify non-toxic variants of these toxins.

It has previously been shown that a NetB mutant (W262A) was able to induce partial protection against experimental NE in poultry [25; WO2013/061056]. The C-terminal region of the alpha-toxin has also been investigated for this purpose (WO93/23543).

### Summary of the Invention

According to a first aspect of the invention, there is provided a composition comprising a reduced toxicity NetB epitope polypeptide and a reduced toxicity *Clostridium perfringens* alpha-toxin epitope polypeptide.

Surprisingly, the present inventors have found that administration of both a reduced toxicity NetB epitope polypeptide and a reduced toxicity *C*. *perfringens* alpha-toxin epitope polypeptide to chickens confers 100% protection against infection by *C. perfringens.* This is unexpected in view of the outcome of administration of *C*. *perfringens* culture supernatants to animals, in which supernatants comprising both wild-type NetB and alpha-toxin varied in their ability to confer protection [21].

The term "NetB epitope polypeptide" as used throughout this specification means a polypeptide which comprises one or more (or all) epitopes of mature wild-type NetB, as represented by the amino acid SEQ ID NO:1. The term *"Clostridium perfringens* alpha-toxin epitope polypeptide" as used throughout this specification means a polypeptide which comprises one or more (or all) epitopes of the *C*. *perfringens* alpha-toxin, as represented by the amino acid SEQ ID NO:18. For example, it may comprise one or more (or all) epitopes of the C-terminal portion of *C*. *perfringens* alpha-toxin, as represented by the amino acid SEQ ID NO:3) (and as disclosed in WO93/23543 as SEQ ID NO:4). The term "epitope" refers to the amino acids (typically a group of around 5 or more amino acids) within a polypeptide sequence which are essential in the generation of an immune response. These amino acids can be consecutive in the sequence but, more typically, are non-consecutive, grouping together when the tertiary structure of the native protein is formed. For example, a NetB epitope polypeptide may be one which is capable of binding to an antibody which binds to the mature wild-type NetB having sequence SEQ ID NO:1, while a *C. perfringens* alpha-toxin epitope polypeptide may be one which is capable of binding to an antibody which binds to *C. perfringens* alpha-toxin, i.e., SEQ ID NO:18 (for example, an antibody which binds to SEQ ID NO:3).

An epitope polypeptide may be, as mentioned above, any which comprises at least one epitope of the polypeptide referred to and is capable of binding an antibody which will bind to the polypeptide referred to, that is, NetB (SEQ ID NO:1) or *C*. *perfringens* alpha-toxin (SEQ ID NO:18). Therefore, the polypeptide may be as little as about 20 amino acids in length provided that it still binds to such an antibody, for example, it may be at least about 30, 40, 50, 60, 70, 80, 90 or 100 amino acids in length. Both the NetB wild-type polypeptide and the alpha-toxin wild-type polypeptide are initially expressed in the cell with a 30- or 28-amino acid N-terminal signal sequence, respectively; the epitope polypeptide may comprise such a signal sequence.

A NetB or alpha-toxin epitope polypeptide being of "reduced toxicity", as referred to throughout this specification, indicates a reduced toxicity as compared to the mature wild-type NetB having sequence SEQ ID NO:1 or mature wild-type alpha-toxin having SEQ ID NO:18, respectively. The level of toxicity may be determined as described herein and in co-pending application PCT/GB2012/052639 (WO2013/061056), for example by use of a chicken hepatocellular carcinoma (LMH) cell-based lactate dehydrogenase (LDH) assay. The level of toxicity should be below that which causes effects in poultry. This can be readily assessed using methods known in the art. In one embodiment, the reduced toxicity is not a function of chemical treatment. In one embodiment, the reduced toxicity is caused by alteration of the polypeptide by substitution, deletion or addition of one or more (i.e. 1, 2, 3, 4, 5, 6, 7, 8, 9, 10 or more) amino acids compared to the wild type sequence.

The NetB epitope polypeptide described herein may comprise a sequence of at least 10 contiguous amino acids from SEQ ID NO:1, the sequence containing at least one amino acid substitution compared to the equivalent sequence of SEQ ID NO:1. For example, the amino acid substitution may be selected from:
a) W262X and W257X, wherein X is any amino acid other than W;
b) Y78X, Y182X, Y187X, Y191X and Y202X, wherein X is any amino acid other than Y;
c) P138X, wherein X is any amino acid other than P;
d) H188X, wherein X is any amino acid other than H; and
e) R200X, wherein X is any amino acid other than R.

The amino acid numbers given above for the substitution positions are with reference to the positions found in SEQ ID NO:1. Where a sequence shorter than SEQ ID NO:1 and comprising at least 10 contiguous amino acids from SEQ ID NO:1 is utilised, the position of the substitution is determined by aligning the rest of the shorter sequence with the sequence of SEQ ID NO:1 so that complete sequence identity is achieved between the aligned sequences, with the exception of the substitution point. Such alignment can readily be achieved using a global sequence alignment program such as the Needleman-Wunsch Global Sequence Alignment Tool, discussed in more detail elsewhere herein.

The amino acid substitutions may be particularly selected from W262X, Y191X, R200X or W257X. In any of the above substitutions, X may be A (alanine). In one embodiment, the substitution is W262A.

In an embodiment described herein, the NetB epitope polypeptide comprises at least one of the sequences:
a) ETTQXRGTNK (SEQ ID NO:10) (for example, the polypeptide may comprise SEQ ID NO:11), wherein X is any amino acid other than W;
b) YHAIXGNQLF (SEQ ID NO:4) (for example, the polypeptide may comprise SEQ ID NO:5), wherein X is any amino acid other than Y;
c) FMKSXLYNNG (SEQ ID NO:6) (for example, the polypeptide may comprise SEQ ID NO:7), wherein X is any amino acid other than R; or
d) YILNXETTQW (SEQ ID NO:8) (for example, the polypeptide may comprise SEQ ID NO:9), wherein X is any amino acid other than W.

Each of these sequences comprises X at a substitution position mentioned above, with SEQ ID NOs:4 and 5 comprising position Y191, SEQ ID NOs:6 and 7 comprising position R200, SEQ ID NOs:8 and 9 comprising W257 and SEQ ID NOs:10 and 11 comprising W262. In an embodiment, X in any of the sequences is A (alanine).

In the composition according to the invention, the NetB epitope polypeptide comprises the amino acid sequence SEQ ID NO:2, or a functional fragment or variant thereof having at least 95% global sequence identity to SEQ ID NO:2 and comprising ETTQARGTNK (SEQ ID NO: 17). SEQ ID NO:2 is the amino acid sequence of NetB comprising substitution W262A. Preparation of this polypeptide is described in co-pending application PCT/GB2012/052639 (WO2013/061056). A functional fragment or variant of SEQ ID NO:2 is a fragment or variant which is capable of binding to an antibody which will bind to wild-type NetB (SEQ ID NO:1) and which has similar toxicity to SEQ ID NO:2, for example assessed by use of a LMH cell-based LDH assay as outlined above. "Similar toxicity" may be, for example, toxicity within about 5%, 10%, 15% or about 20% of the toxicity of SEQ ID NO:2 itself. The functional fragment or variant may have global sequence identity, for example assessed using the Needleman-Wunsch Global Sequence Alignment Tool described elsewhere herein, of at least about 95%, 96%, 97%, 98% or at least about 99%. For example, the functional fragment may have up to 73 amino acids removed from the N- or C-terminal ends of the polypeptide, for example, up to about 10, 20, 30, 40, 50, 60 or about 70 amino acids removed. This figure may be the total number of amino acids removed from the polypeptide, with some of the total number removed from the C-terminal end and some of the total number removed from the N-terminal end. The functional fragment or variant comprises the amino acid sequence ETTQARGTNK (SEQ ID NO:17), which is a ten amino acid sequence comprising amino acid A at the position equivalent to position 262 of the full length polypeptide (SEQ ID NO:2) when SEQ ID NOs:2 and 17 are aligned. In one embodiment, a variant of a NetB epitope polypeptide has one or more (i.e. 1, 2, 3, 4, 5, 6, 7, 8, 9, 10 or more) amino acid substitutions, deletions or additions when compared to SEQ ID NO:2.

In this application, the alpha-toxin epitope polypeptide may comprise a sequence of at least 5 contiguous amino acids from SEQ ID NO:18. For example, it may comprise a sequence of at least 5 contiguous amino acids from SEQ ID NO:3, the sequence containing sequences DDY and/or PGN. SEQ ID NO:3 is the amino acid sequence of the C-terminal region (residues 247-370) of the alpha-toxin polypeptide disclosed as SEQ ID NO:2 in WO93/23542. The residues 273-275 (DDY) and 295-297 (PGN) of the full-length polypeptide have previously been identified as the positions of some of the C-terminal epitopes (WO93/23543; Logan et al. (1991) Infect. Immun. vol. 59, p 4338-4342).

For example, the alpha-toxin epitope polypeptide may comprise the amino acid sequence SEQ ID NOs:12 and/or 13 and/or 14 and/or 15. SEQ ID NOs:12 and 13 are found in SEQ ID NO:3 and comprise the DDY position; SEQ ID NOs: 14 and 15 are found in SEQ ID NO:3 and comprise the PGN position. The alpha-toxin epitope polypeptide of the invention comprises the amino acid sequence SEQ ID NO:16; this is found in SEQ ID NO:3 and comprises the DDY position and the PGN position and all of SEQ ID NOs: 12-15.

In an embodiment, the alpha-toxin epitope polypeptide comprises the amino acid sequence SEQ ID NO: 18, or a functional fragment or variant thereof having at least 34% global sequence identity to SEQ ID NO: 18. A functional fragment or variant of SEQ ID NO: 18 is a fragment or variant which is capable of binding to an antibody which will bind to SEQ ID NO:18. The functional fragment or variant may have global sequence identity, for example assessed using the Needleman-Wunsch Global Sequence Alignment Tool described elsewhere herein, of at least about 95%, 96%, 97%, 98% or about 99%. For example, the functional fragment may have up to 246 amino acids removed from one of the ends of the polypeptide.

For example, amino acid residues 1-246 may be removed from the N-terminal end of SEQ ID NO:18, with residues 247-370 remaining as SEQ ID NO:3 (which, at a global level, is 34% identical to SEQ ID NO:18), which may form the alpha-toxin epitope polypeptide referred to herein. In the composition according to the invention, the alpha-toxin epitope polypeptide comprises the amino acid sequence SEQ ID NO:3, or comprises a functional fragment or variant of SEQ ID NO:3, having at least 95% global sequence identity thereto and comprises the amino acid sequence of SEQ ID NO: 16. A functional fragment or variant of SEQ ID NO:3 is a fragment or variant which is capable of binding to an antibody which will bind to SEQ ID NO:3. The functional fragment or variant may have global sequence identity, for example assessed using the Needleman-Wunsch Global Sequence Alignment Tool described elsewhere herein, of at least about 95%, 96%, 97%, 98% or about 99%. For example, the functional fragment may have up to 31 amino acids removed from the N- or C-terminal ends of the polypeptide, for example, up to about 5, 10, 15, 20, 25 or about 30 amino acids removed. This figure may be the total number of amino acids which are removed from the polypeptide, with some of the total number removed from the C-terminal end and some of the total number removed from the N-terminal end. In one embodiment, a variant of an alpha-toxin epitope polypeptide has one or more (i.e. 1, 2, 3, 4, 5, 6, 7, 8, 9, 10 or more) amino acid substitutions, deletions or additions when compared to SEQ ID NO:18.

As mentioned, the present disclosure encompasses a composition comprising variants of the epitope polypeptides and methods utilising these variant polypeptides. As used herein, a "variant" means a polypeptide in which the amino acid sequence differs from the base sequence from which it is derived in that one or more amino acids (i.e. 1, 2, 3, 4, 5, 6, 7, 8, 9, 10 or more) within the sequence are substituted for other amino acids, or are deleted or added. The variant is a functional variant, in that the functional characteristics of the polypeptide from which the variant is derived are maintained. For example, the variant polypeptide may have a similar ability to bind an antibody capable of binding to a non-variant polypeptide (such as SEQ ID NO:1, 18 or 3). In particular, any amino acid substitutions, additions or deletions must not alter or significantly alter the tertiary structure of one or more epitopes contained within the polypeptide from which the variant is derived, so that the variant polypeptide retains the ability to bind to an antibody which binds to SEQ ID NO:1, 18 or 3, as appropriate. The skilled person is readily able to determine appropriate functional variants and to determine the tertiary structure of an epitope and any alterations thereof, without the application of inventive skill. Amino acid substitutions may be regarded as "conservative" where an amino acid is replaced with a different amino acid with broadly similar properties. Non-conservative substitutions are where amino acids are replaced with amino acids of a different type.

By "conservative substitution" is meant the substitution of an amino acid by another amino acid of the same class, in which the classes are defined as follows:

| Class | Amino acid examples |
|---|---|
| Nonpolar: | Ala, Val, Leu, Ile, Pro, Met, Phe, Trp |
| Uncharged polar: | Gly, Ser, Thr, Cys, Tyr, Asn, Gln |
| Acidic: | Asp, Glu |
| Basic: | Lys, Arg, His. |

As is well known to the skilled person, altering the primary structure of a polypeptide by a conservative substitution may not significantly alter the activity of that polypeptide because the side-chain of the amino acid which is inserted into the sequence may be able to form similar bonds and contacts as the side chain of the amino acid which has been substituted out. This is so even when the substitution is in a region which is critical in determining the polypeptide's conformation.

As mentioned above, non-conservative substitutions are possible provided that these do not disrupt the tertiary structure of an epitope within the polypeptide, for example, which do not interrupt the immunogenicity (for example, the antigenicity) of the polypeptide.

Broadly speaking, fewer non-conservative substitutions will be possible without altering the biological activity of the polypeptide. As mentioned above, variants may suitably be at least about 75% identical to the base sequence.

Sequence identity between amino acid sequences can be determined by comparing an alignment of the sequences. When an equivalent position in the compared sequences is occupied by the same amino acid, then the molecules are identical at that position. Scoring an alignment as a percentage of identity is a function of the number of identical amino acids at positions shared by the compared sequences. When comparing sequences, optimal alignments may require gaps to be introduced into one or more of the sequences, to take into consideration possible insertions and deletions in the sequences. Sequence comparison methods may employ gap penalties so that, for the same number of identical molecules in sequences being compared, a sequence alignment with as few gaps as possible, reflecting higher relatedness between the two compared sequences, will achieve a higher score than one with many gaps. Calculation of maximum percent identity involves the production of an optimal alignment, taking into consideration gap penalties.

As mentioned previously, sequence identity preferably is determined using the Needleman-Wunsch Global Sequence Alignment Tool available from the National Center for Biotechnology Information (NCBI), Bethesda, Maryland, USA, for example via http://blast.ncbi.nlm.nih.gov/Blast.cgi, using default parameter settings. When comparing the level of sequence identity to SEQ ID NO:1, for example, this typically should be done relative to the whole length of SEQ ID NO:1, to avoid short regions of high identity overlap resulting in a high overall assessment of identity (i.e., a global alignment method is used).

In the composition according to the invention, at least one polypeptide is a recombinant polypeptide. That is, the polypeptide is not naturally occurring, but has been prepared using established laboratory techniques, as outlined, for example, in Green & Sambrook (Molecular Cloning, a laboratory manual [fourth edition] Green & Sambrook, Cold Spring Harbor Laboratory, 2012). Such techniques are well known to the skilled person.

Polypeptides used with the disclosure are preferably provided in purified or substantially purified form *i.e.* substantially free from other polypeptides (*e.g.* free from naturally-occurring polypeptides), particularly from other *Clostridium* or host cell polypeptides, and are generally at least about 50% pure (by weight), and usually at least about 90% pure *i.e.* less than about 50%, and more preferably less than about 10% (*e.g.* 5%) of a composition is made up of other expressed polypeptides. Thus the polypeptides in the compositions are separated from the whole organism with which the molecule is expressed.

The alpha-toxin epitope polypeptide may be expressed as a fusion protein with Glutathione-S-Transferase (GST) or with a Histidine tag (His-tag). The NetB epitope polypeptide may be expressed with a Histidine tag (His-tag) or a fusion protein with Glutathione-S-Transferase (GST). Other expression tags may readily be utilised by the skilled person without application of inventive skill.

The composition according to the invention may comprise a fusion protein comprising a reduced toxicity NetB epitope polypeptide as described above and a reduced toxicity C. *perfringens* alpha-toxin epitope polypeptide as described above. Optionally, such a fusion protein may comprise a linker sequence between the two epitope polypeptides. Suitable linker amino acid sequences will be apparent to those skilled in the art, but include poly-glycine linkers (*i.e.* comprising Gly*ₙ* where *n* = 2, 3, 4, 5, 6, 7, 8, 9, 10 or more), and GSGGGG (SEQ ID NO: 21), with the Gly-Ser dipeptide being formed from a *Bam*HI restriction site, thus aiding cloning and manipulation, and the (Gly)₄ tetrapeptide being a typical poly-glycine linker. The composition may be a simple mixture of polypeptides in solution, for example in a buffer solution, or may form part of a composition comprising other components, such as in a vaccine composition as described below.

According to a second aspect of the invention, there is provided a polynucleotide encoding a reduced toxicity NetB epitope polypeptide and a reduced toxicity *C. perfringens* alpha-toxin epitope polypeptide. Therefore, a single polynucleotide sequence comprises a polynucleotide encoding for each polypeptide; these polynucleotides may be immediately adjacent one another or may be separated by a further polynucleotide sequence not encoding either polypeptide. The polynucleotide may be contained within a vector, which forms a third aspect of the invention. The vector may comprise additional expression control sequences such as at least one promoter sequence operably linked to the sequence(s) encoding for the polypeptides, terminator region and one or more inducer regions. The polynucleotide encoding the NetB epitope polypeptide may be under the control of a separate promoter region to the polynucleotide encoding the alpha-toxin epitope polypeptide. The design of a suitable vector is within the routine abilities of the skilled person, for example, with reference to Green & Sambrook (Molecular Cloning, a laboratory manual [fourth edition] Green & Sambrook, Cold Spring Harbor Laboratory, 2012).

A fourth aspect of the invention provides a cell comprising a composition according to the first aspect, a polynucleotide according to the second aspect and/or a vector according to the third aspect of the invention. For example, a suitable cell may be a *Salmonella* cell, such as a *Salmonella enterica* cell, in some embodiments from the serovar *typhimurium.* The *Salmonella* may be an attenuated strain. Strains χ8914 and χ9241 may optionally be employed. Such cells are particularly useful to act as vectors when the polypeptide, polynucleotide and vector of the invention is to be used to provide a vaccine for chickens, to reduce the probability that they will be susceptible to infection by *C. perfringens.* For example, such a system is described in Kulkarni *et al.* [33].

Other suitable cells may include attenuated mutants of Bacillus species or of *C*. *perfringens.* Inclusion of the polypeptides in a bacterium that is normally a member of the subject's gut flora is also contemplated. *Lactococcus* and *Lactobacillus* species are also contemplated.

A fifth aspect of the invention provides a vaccine composition comprising a composition according to the first aspect and/or a polynucleotide according to the second aspect and/or a vector according to the third aspect and/or a cell according to the fourth aspect of the invention. The vaccine composition may comprise a subunit vaccine, for example, in the form of a fusion protein and/or a recombinant viral vaccine.

The vaccine composition may further comprise excipients and/or diluents appropriate for the means by which the composition is to be administered to a subject in need of vaccination against infection by C. *perfringens.* Selection of appropriate components is within the routine capability of the skilled person without the application of inventive activity.

For example, the vaccine composition of the invention may conveniently be formulated using a pharmaceutically acceptable excipient or diluent, such as, for example, an aqueous solvent, non-aqueous solvent, non-toxic excipient, such as a salt, preservative, buffer and the like. Examples of non-aqueous solvents are propylene glycol, polyethylene glycol, vegetable oil and injectable organic esters such as ethyloleate. Aqueous solvents include water, alcoholic/aqueous solutions, saline solutions, parenteral vehicles such as sodium chloride, Ringer's dextrose, etc. Preservatives include antimicrobials, anti-oxidants, chelating agents and inert gases. The pH and exact concentration of the various components the vaccine composition are adjusted according to routine skills.

Optionally, the vaccine formulation may include a carrier. Commonly used carrier molecules are bovine serum albumin (BSA), keyhole limpet hemocyanin (KLH), ovalbumin, mouse serum albumin, rabbit serum albumin and the like. Synthetic carriers may be used and are readily available. Means for conjugating peptides to carrier proteins are well known in the art and include glutaraldehyde, m-maleimidobenzoyl-N-hydroxysuccinimide ester, carbodiimide and bis-biazotized benzidine.

In certain situations, it may also be desirable to formulate the vaccine composition to comprise an adjuvant to enhance the immune response. Such adjuvants include all acceptable immunostimulatory compounds such as, for example, a cytokine, toxin, or synthetic composition. Commonly used adjuvants include the mineral-containing compositions aluminium hydroxide, aluminium phosphate, calcium phosphate; oil emulsions including squalene-water emulsions such as MF59, complete and incomplete Freund's adjuvants, and the Montanide ISA series of animal adjuvants; saponin formulations including Quil-A saponin, such as QS21 marketed as Stimulon™, as well as lipid formulations, such as ISCOMs; bacterial or microbial derivatives, such as non-toxic derivatives of enterobacterial lipopolysaccharide (LPS) including monophosphoryl lipid A (MPL) and 3-O-deacylated MPL (3dMPL); polymer adjuvants such as Montanide gel; as well as combinations thereof such as AS04 (comprising aluminium phosphate and MPL). In addition to adjuvants, it may be desirable to co-administer biologic response modifiers (BRM) with the peptide or variant or derivative to down regulate suppressor T cell activity.

Possible vehicles for administration of the vaccine formulation include liposomes. Liposomes are microscopic vesicles that consist of one or more lipid bilayers surrounding aqueous compartments. Liposomes are similar in composition to cellular membranes and, as a result, liposomes generally can be administered safely and are biodegradable. Techniques for preparation of liposomes and the formulation (e.g., encapsulation) of various molecules, including peptides and oligonucleotides, with liposomes are well known.

Depending on the method of preparation, liposomes may be unilamellar or multilamellar and can vary in size with diameters ranging from 0.02µm to greater than 10µm. Liposomes can also adsorb to virtually any type of cell and then release the encapsulated agent. Alternatively, the liposome fuses with the target cell, whereby the contents of the liposome empty into the target cell. Alternatively, an absorbed liposome may be endocytosed by cells that are phagocytic. Endocytosis is followed by intralysosomal degradation of liposomal lipids and release of the encapsulated agents. In the present context, the polypeptides in the composition according to the first aspect of the invention can be localized on the surface of the liposome, to facilitate antigen presentation without disruption of the liposome or endocytosis. Irrespective of the mechanism or delivery, however, the result is the intracellular disposition of the associated polypeptides.

Liposomal vectors may be anionic or cationic. Anionic liposomal vectors include pH sensitive liposomes which disrupt or fuse with the endosomal membrane following endocytosis and endosome acidification. Cationic liposomes are preferred for mediating mammalian cell transfection *in vitro,* or general delivery of nucleic acids, but are used for delivery of other therapeutics, such as peptides.

Other suitable liposomes that are used in the compositions and methods of the invention include multilamellar vesicles (MLV), oligolamellar vesicles (OLV), unilamellar vesicles (UV), small unilamellar vesicles (SUV), medium-sized unilamellar vesicles (MIN), large unilamellar vesicles (LUV), giant unilamellar vesicles (GUV), multivesicular vesicles (MVV), single or oligolamellar vesicles made by reverse-phase evaporation method (REV), multilamellar vesicles made by the reverse-phase evaporation method (MLV-REV), stable plurilamellar vesicles (SPLV), frozen and thawed MLV (FATMLV), vesicles prepared by extrusion methods (VET), vesicles prepared by French press (FPV), vesicles prepared by fusion (FUV), dehydration-rehydration vesicles (DRV), and bubblesomes (BSV). Techniques for preparing these liposomes are well known in the art.

Other forms of delivery particle, for example, microspheres and the like, also are contemplated for delivery of the epitope polypeptides.

Alternatively, nucleic acid-based vaccines may be produced that comprise nucleic acid, such as, for example, DNA or RNA, encoding the immunologically active epitope polypeptides and cloned into a suitable vector (e.g., vaccinia, canarypox, adenovirus, or other eukaryotic virus vector).

Compositions of the invention may be administered as a liquid, emulsion, dried powder and/or in a mist. Such compositions may be administered parenterally or enterally. If parenteral administration is desired, the compositions may be injected subcutaneously, intramuscularly, intraperitoneally or intradermally, or alternatively may be delivered by scarification or via a mucosal route such as intranasally, by aerosol or eye drop.

When intended for use with birds, the vaccine composition may be formulated to be suitable for *in ovo* administration, for example, via injection into the egg prior to hatching (e.g., into the amniotic fluid, the body of the embryo or the yolk sac). For example, injection into the amniotic fluid may be followed by ingestion by the embryo prior to hatching.

Alternatively, the composition may take the form of a cellular vaccine for delivery via the administration of autologous or allogeneic APCs or dendritic cells that have been treated *in vitro* so as to present the polypeptides on their surface. *Salmonella* cells may also be used, especially for administration to poultry, such as chickens. This involves the use of live attenuated *Salmonella* vaccines to deliver the antigens. This approach offers a number of advantages. First, live *Salmonella* vaccines can be given orally (the natural route of infection), enabling a non-invasive route of vaccine administration. Second, both mucosal and systemic immune responses can be elicited, which may be important for protection against infection. In addition, live attenuated *Salmonella* vaccines are able to simulate both humoral and cellular immune responses that may be important for protection against disease. Finally, since *Salmonella* is genetically tractable, recombinant *Salmonella* vaccines are relatively easy to develop and are also relatively cost effective to produce.

One of the most widely studied classes of attenuated *Salmonella* used as carriers of foreign antigens are auxotrophs. For example, genetically defined mutants of the *aroA* gene, encoding 5-*enol*pyruvylshikimate-3-phosphate synthase, have been constructed in both *S. enterica* var. Typhimurium and var. Typhi. These mutants are attenuated and immunogenic in mice. Examples of other auxotrophic mutants include *Salmonella* with deletions in the genes involved in the purine biosynthetic pathway. Another well-studied group of attenuated *Salmonella* are mutants that have defined deletions in genes involved in the regulation of *Salmonella* virulence. For example, mutations in genes encoding adenylate cyclase (*cya*) and camp receptor protein (*crp*) affect the expression of genes involved.

Other suitable cells may include attenuated mutants of Bacillus species or of *C*. *perfringens.* Inclusion of the polypeptides in a bacterium that is normally a member of the subject's gut flora is also contemplated. *Lactococcus* and *Lactobacillus* species are also contemplated.

In one embodiment, the vaccine composition may be included in an animal feed (i.e., a foodstuff suitable for consumption by an animal, particularly a chicken) comprising a composition and/or a polynucleotide and/or a vector and/or a cell and/or vaccine composition according to preceding aspects of the invention. This may, in non-limiting examples, be in the form of pellets, crumbs or a mash which may further comprise, again for example only, grain, grass and/or protein components. The composition may also be included in drinking liquids and/or administered via a spray into the atmosphere surrounding the animal which is, consequently, inhaled by the animal.

In a sixth aspect of the invention, there is provided a composition according to the first aspect, or a polynucleotide according the second aspect, or a vector according to the third aspect, or a cell according to the fourth aspect, or a vaccine composition according to the fifth aspect, for use in (i) therapy, (ii) eliciting an immune response against *C*. *perfringens,* and/or (iii) a method of vaccinating a subject against infection by *Clostridium perfringens.*

Likewise, a seventh aspect of the invention provides a method of (i) eliciting an immune response against *C*. *perfringens* and/or (ii) vaccinating a subject against infection by *C*. *perfringens* comprising administering to the subject a protective amount of a composition according to the first aspect, or a polynucleotide according to the second aspect, or a vector according to the third aspect, or a cell according to the fourth aspect, or a vaccine composition according to the fifth aspect of the invention.

A related eighth aspect of the invention providing a method of (i) eliciting an immune response against *C. perfringens* and/or (ii) vaccinating a subject against infection by *C*. *perfringens* may comprise administering to the subject a protective amount of a reduced toxicity NetB epitope polypeptide in combination with a protective amount of a reduced toxicity *C*. *perfringens* alpha-toxin epitope polypeptide. In the context of this aspect of the invention, the term "protective amount" of each polypeptide indicates an amount which is effective to vaccinate the subject against infection by *C*. *perfringens* when administered in combination with the other polypeptide. A related ninth aspect of the invention provides a reduced toxicity NetB epitope polypeptide in combination with a reduced toxicity *C*. *perfringens* alpha-toxin epitope polypeptide, for use in a method of vaccinating a subject against infection by *C. perfringens.* In such combinations, the component polypeptides may be administered simultaneously (i.e. by way of the same delivery device, or in the same feed) or sequentially (i.e. one after the other either using the same or different delivery devices/feeds). If administered sequentially, the polypeptides are preferably administered within about 24 hours or less (i.e. 18 hours, 12 hours, 6 hours, 3 hours, 1 hour, 45 minutes, 30 minutes, 15 minutes, 10 minutes, 5 minutes or less) of each other.

In both the seventh and eighth aspects, the term "protective amount" indicates an amount sufficient to induce an immune response in the subject, such that the probability of the subject becoming infected by *C*. *perfringens* if exposed to the bacterium is reduced or removed. For example, antibodies capable of binding to SEQ ID NO:1 and antibodies capable of binding to SEQ ID NO:18 and/or 3 may be detectable after the administration, where such antibodies were not detectable prior to the administration, or only detectable at lower concentrations than after administration.

In the sixth, seventh, eighth and ninth aspects, the subject may be poultry, such as chicken, turkey or duck. In one embodiment, the subject is of the genus *Gallus*, for example, of the species *Gallus gallus* (i.e., the domestic chicken). When the subject is a chicken, the preferred means for delivery of the polypeptide and/or a polynucleotide and/or a vector and/or a cell and/or a subunit vaccine of the other aspects of the invention may be a *Salmonella*-based system as described herein. The subject may also be a mammalian subject, for example, a human.

The term "vaccinating a subject", as used herein, may indicate that the subject is completely protected from becoming infected by *C. perfringens.* That is, in any population vaccinated according to the described methods and/or using the described compositions or kits, less than 10%, preferably less than 5%, preferably 0% of individual subjects within that population will become infected by *C*. *perfringens* after exposure thereto. Infection may be assessed, for example, at least about 1, 2, 3, 4, 5, 6, 7, 10, 15, 21 or at least about 28 days after exposure of the subject to *C*. *perfringens.* One method of assessing infection is by using scoring lesions within the small intestine of each animal, as described by Keyburn *et al.* [29]. A non-invasive method of assessing infection is by monitoring the weight gain of each animal, wherein slower weight gain of a particular animal when compared to other similarly aged animals is indicative of infection. In one embodiment, the invention also provides a method of providing immunity to the progeny of a female mammal, comprising administering an effective amount of a composition of the invention to the female mammal prior to the birth of her progeny, whereby upon birth the progeny have acquired passive immunity to *C. perfringens* infection.

According to a tenth aspect of the invention, there is provided a kit comprising a composition and/or a polynucleotide and/or a vector and/or a cell and/or a vaccine composition according to any of the preceding aspects. In a related aspect, a kit may comprise a reduced toxicity NetB epitope polypeptide and a reduced toxicity *C*. *perfringens* alpha-toxin epitope polypeptide. For example, the kit may be a kit for use by a veterinarian or farmer to vaccinate a flock of chickens and may comprise a cellular vector (e.g., *Salmonella* or a Bacillus species etc., as described above) comprising a polypeptide according to the invention, for example for administration to chickens by inclusion in their feed. Instructions for use of the kit may be enclosed therewith. The kit may comprise a vial containing a composition and/or a polynucleotide and/or a vector and/or a cell and/or a vaccine composition according to any of the preceding aspects. The kit may also comprise a device for administration, for example, a syringe.

Throughout the description and claims of this specification, the words "comprise" and "contain" and variations of the words, for example "comprising" and "comprises", mean "including but not limited to" and do not exclude other moieties, additives, components, integers or steps. The term "about" in relation to a numerical value *x* means, for example, *x*±10%.

Throughout the description and claims of this specification, the singular encompasses the plural unless the context otherwise requires. In particular, where the indefinite article is used, the specification is to be understood as contemplating plurality as well as singularity, unless the context requires otherwise.

Other features of the present invention will become apparent from the following examples. Generally speaking the invention extends to any novel one, or any novel combination, of the features disclosed in this specification (including the accompanying claims and drawings). Thus, features, integers, characteristics, compounds or chemical moieties described in conjunction with a particular aspect, embodiment or example of the invention are to be understood to be applicable to any other aspect, embodiment or example described herein, unless incompatible therewith.

Moreover, unless stated otherwise, any feature disclosed herein may be replaced by an alternative feature serving the same or a similar purpose.

### Brief Description of the Figures

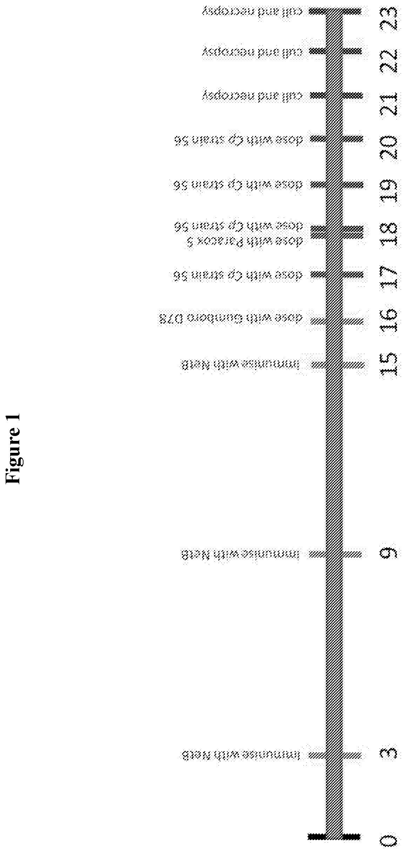

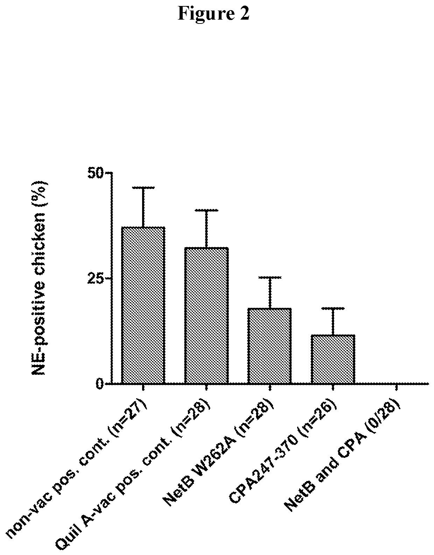

The invention will now be described, by way of example only, with reference to Figures 1 and 2 in which:
Figure 1 is a schematic outline of the NE experimental design, with animals immunised with formaldehyde NetB toxoid or NetB W262A on days 3, 9, and 15 post-hatching and infected with *C*. *perfringens* strain 56 on days 17, 18, 19, and 20; animals were culled, necropsied, and scored for lesions within the small intestines on days 21, 22, or 23; and
Figure 2 shows the lesion scores for poultry experimentally infected with *C. perfringens*, with control groups of untreated chickens or chickens dosed with adjuvant only, chickens immunised with NetB W262A or the alpha-toxin CPA247-370 alone and chickens immunised with a combination of NetB W262A and CPA247-370.

### Examples

### Materials and methods

### Bacterial strains and plasmids

Plasmid pBAD (Invitrogen, Paisley, UK) was used as expression vector and *Eschericia coli* TOP10 (Invitrogen, Paisley, UK) as expression host. *E. coli* strains were grown either in Terrific Broth (TB) or Luria-Bertani (LB) agar supplemented with ampicillin (100 µg/ml) at 37°C and shaken at 300 rpm, where appropriate.

The formulation of TB and LB was:

### TB:

12g tryptone
24g yeast extract
4ml glycerol
Adjust volume to 1000ml with 100ml of a filter sterilized solution of 0.17M KH₂PO₄ and 0.72M K₂HPO₄

### LB:

10g Bacto-tryptone
5g yeast extract
10g NaCl
Adjust pH to 7.5 with NaOH.
Adjust volume to 1000ml with dH₂O

### Animals and housing conditions

Ross 308 broiler chickens were obtained as one-day-old chicks from Vervaeke-Belavi Hatchery (Tielt, Belgium, BE3031) and the parent flock had not been vaccinated with the commercial Netvax or any other *C*. *perfringens* vaccine. All animals were housed in the same room. The birds were reared in pens at a density of 26-28 animals per 1.5 m² on wood shavings. All pens were separated by solid walls to prevent contact between birds from different treatment groups. Before the trial, the rooms were decontaminated with Metatectyl HQ (Clim'oMedic®, Metatecta, Belgium) and a commercial anticoccidial disinfectant (OOCIDE, DuPont Animal Health Solutions, Wilmington, USA). The chickens received *ad libitum* drinking water and feed. A 23 h/l h light/darkness program was applied. The animal experiments were carried out according to the recommendations and following approval of the Ethical Committee of the Faculty of Veterinary Medicine, Ghent University, Belgium.

### Expression and purification ofNetB W262A

The expression and purification of NetB W262A with N-terminal His-tags (SEQ ID NO:19) for purification, was carried out in *E. coli* TOP10 cells and purification was carried out as described previously [25]. In summary, the recombinant *E. coli* carrying the pBAD-NetB expression vector was grown in TB to an optical density (OD₅₉₅ₙₘ) of 0.5 and expression of the toxin induced for 6 h by adding arabinose at a final concentration of 0.02% (w/v). Bacterial cells were harvested by centrifugation, lysed enzymatically using BugBuster (Invitrogen, Paisley, UK), and NetB purified by Ni-NTA chromatography columns (GE Healthcare Life Sciences, Little Chalfont, UK) according to the manufacturer's instructions. The protein was transferred into Tris-buffered saline (TBS; 20 mM Tris pH 7.5, 150 mM NaCl) using PD-10 desalting columns (GE Healthcare Life Sciences, Little Chalfont, UK) and protein concentrations measured with a UV-Vis spectrophotometer (Thermo Scientific, Cramlington, UK).

### Expression and purification of CPA247-370

Alpha-toxoid was produced and purified as described previously [26]. In brief, recombinant *E. coli* containing the plasmid pGEX-3X-13, which encodes the C-terminal domain of the alpha-toxin fused to GST (GST-Cpa₂₄₇₋₃₇₀; SEQ ID NO:20), was used. Bacteria were cultured in TB medium (100 µg/ml ampicillin) under gentle rotation at 37°C. Expression of alpha-toxoid was induced at an OD₅₉₅ₙₘ of 0.5 for 6h by the addition of IPTG (ImM final concentration). Bacterial cells were harvested by centrifugation, lysed enzymatically using BugBuster (Invitrogen, Paisley, UK), and GST-Cpa₂₄₇₋₃₇₀ purified with prepacked GST GraviTrap columns (GE Healthcare Life Sciences, Little Chalfont, UK) according to the manufacturer's instructions. The eluted protein was dialysed against TBS by using PD-10 desalting columns (GE Healthcare Life Sciences, Little Chalfont, UK).

### In vivo NE model

The NE model was based on the subclinical *in vivo* model described previously [28]. On days 3, 9, and 15, chickens were each immunised with 30 µg of either NetB W262A or CPA247-370 or both. Quil-A (50 µg; Brenntag Biosector, Frederikssund, Denmark) was used as an adjuvant. The mixture was diluted in PBS to a total volume of 200 µl, mixed well by vortexing and filter-sterilised (0.2 µm pore size). Birds were vaccinated subcutaneously in the neck with a 200 µl dose. Controls consisted of an untreated group and a group receiving only the Quil-A (50 µg) adjuvant. Sera samples were taken on day 16 and on days 21, 22 and 23.

Nobilis Gumboro D 78 vaccine (Schering-Plough Animal Health, Brussels, Belgium) was given in the drinking water on day 16. From day 17 onwards, soy bean meal was replaced by fishmeal (30%) as a protein source. All groups were challenged orally, using a plastic tube inserted in the crop, on days 17, 18, 19 and 20 with a single dose of approximately 4 x 10⁸ cfu of *C. perfringens* strain 56 [25]. On day 18, all animals were orally inoculated with a 10 x dose of Paracox-5 (Schering-Plough Animal Health, Brussels, Belgium) [28]. On days 21, 22, and 23, one-third of the birds in each group were euthanised and necropsied. A schematic outline of the experimental design is shown in Figure 1.

### Measurement of antibody to NetB and alpha-toxin using ELISA

Sera samples were taken on day 16 and on day 21-22-23.

### Assessment of protection

NE severity was assessed by scoring lesions within the small intestine of each animal (duodenum to ileum) as described by Keyburn *et al.* [29] as follows: 0 = no gross lesions; 1 = congested intestinal mucosa; 2 = focal necrosis or ulceration (1-5 foci); 3 = focal necrosis or ulceration (6-15 foci); 4 = focal necrosis or ulceration (≥16 foci); 5 = patches of necrosis 2-3 cm long; 6 = diffuse necrosis typical of field cases. Animals showing lesion scores of 2 or higher were classified as NE positive.

### Statistical analysis

For the *in vivo* NE model, differences within the occurrence of NE-positive animals between the controls and the NetB toxoid vaccinated groups were evaluated by a binary logistic regression analysis with the SPSS Statistics software 21.0 (SPSS Inc., Chicago, USA). A 2-way ANOVA analysis with the GraphPad Prism software 5.01 (GraphPad Software, La Jolla, USA) was used to compare the means of ELISA data. In both analysis, a p value of less than 0.01 was considered as significant (***: p<0.001; **: p<0.01).

### Cytotoxicity assay for effect of rNetB on LMH cells

Cytotoxicity is measured using the CytoTox96® kit (Promega), in which cytotoxicity is measured as the amount of LDH (lactate dehydrogenase) released from the cell cytosol into the medium caused by the presence of the tested pore-forming cytotoxic compound. A polypeptide is evaluated for its cytotoxicity by incubation with chicken hepatocellular carcinoma (LMH; ATTC: CRL-2117) cells, a cell line known to be susceptible for the toxin.

LMH cells are grown in Waymouth's MB 752/1 medium (Invitrogen) supplemented with 10% fetal calf serum at 37°C in a 5% CO₂ incubator to 70-80% confluency in 96-well plates. Cells are incubated with serial dilutions of NetB in Waymouth's medium (100µl final volume in each well) for 2h at 37°C. Control cells are incubated with Waymouth's medium to determine either the base line (0%) or total cell lysis (100%), achieved by freezing and thawing of the cells. After 2h of incubation the supernatant is assayed and percentage cytotoxicity was determined relative to the control groups.

### Results

*Protection against experimental NE after immunisation with NetB W262A and CPA247-370* Immunisation with NetB W262A and CPA247-370 reduced lesion scores completely to the control groups in poultry experimentally infected with *C. perfringens.* In the control groups of untreated chickens, or chickens dosed with adjuvant only, the percentage positive chickens were 37% and 32%, respectively. In contrast, in the group immunised with NetB W262A 18% of the chickens showed lesions and in animals immunised with CPA247-370 12% of the chickens were NE-positive. None of the animals vaccinated with a combination of NetB W262A and CPA247-370 developed NE lesions (Figure 2).

### References

[1] Songer JG. Clostridial enteric diseases of domestic animals. Clin Microbiol Rev 1996;9(2):216-34.
[2] McDonel JL. Clostridium perfringens toxins (type A, B, C, D, E). Pharmacol Ther 1980;10(3):617-55.
[3] Petit L, Gibert M, Popoff MR. Clostridium perfringens: toxinotype and genotype. Trends Microbiol 1999;7(3):104-10.
[4] Cooper KK, Songer JG. Virulence of Clostridium perfringens in an experimental model of poultry necrotic enteritis. Vet Microbiol 2009;142(3-4):323-8.
[5] Parish WE. Necrotic enteritis in the fowl (Gallus gallus domesticus). I. Histopathology of the disease and isolation of a strain of Clostridium welchii. J Comp Pathol 1961;71:377-93.
[6] Keyburn AL, Boyce JD, Vaz P, Bannam TL, Ford ME, Parker D, et al. NetB, a new toxin that is associated with avian necrotic enteritis caused by Clostridium perfringens. PLoS Pathog 2008 Feb 8;4(2):e26.
[7] Sluis Vd. Clostridial enteritis is an often underestimated problem. World Poultry 2000;16:42-3.
[8] Castanon JI. History of the use of antibiotic as growth promoters in European poultry feeds. Poult Sci 2007;86(11):2466-71.
[9] Kaldhusdal M, Hofshagen M. Barley inclusion and avoparcin supplementation in broiler diets. 2. Clinical, pathological, and bacteriological findings in a mild form of necrotic enteritis. Poul Sci 1992;71:1145-53.
[10] Brennan J, Moore G, Poe SE, Zimmermann A, Vessie G, Barnum DA, et al. Efficacy of in-feed tylosin phosphate for the treatment of necrotic enteritis in broiler chickens. Poult Sci 2001 Oct;80(10):1451-4.
[11] Brennan J, Bagg R, Barnum D, Wilson J, Dick P. Efficacy of narasin in the prevention of necrotic enteritis in broiler chickens. Avian Dis 2001 Jan-Mar;45(1):210-4.
[12] Elwinger K, Schneitz C, Berndtson E, Fossum O, Teglof B, Engstom B. Factors affecting the incidence of necrotic enteritis, caecal carriage of Clostridium perfringens and bird performance in broiler chicks. Acta Vet Scand 1992;33(4):369-78.
[13] Kaldhusdal M, Schneitz C, Hofshagen M, Skjerv E. Reduced incidence of Clostridium perfringens-associated lesions and improved performance in broiler chickens treated with normal intestinal bacteria from adult fowl. Avian Dis 2001 Jan-Mar;45(1):149-56.
[14] Dahiya JP, Wilkie DC, Van Kessel AG, Drew MD. Potential strategies for controlling necrotic enteritis in broiler chickens in post-antibiotic era. Animal Feed Sci Technol 2006;129:60-88.
[15] Shojadoost B, Vince AR, Prescott JF. The successful experimental induction of necrotic enteritis in chickens by Clostridium perfringens: a critical review. Vet Res 2012 Oct 26;43(1):74.
[16] Chalmers G, Bruce HL, Hunter DB, Parreira VR, Kulkarni RR, Jiang YF, et al. Multilocus sequence typing analysis of Clostridium perfringens isolates from necrotic enteritis outbreaks in broiler chicken populations. J Clin Microbiol 2008;46(12):3957-64.
[17] Martin TG, Smyth JA. Prevalence of netB among some clinical isolates of Clostridium perfringens from animals in the United States. Vet Microbiol 2009;136(1-2):202-5.
[18] Savva CG, Fernandes da Costa SP, Bokori-Brown M, Naylor CE, Cole AR, Moss DS, et al. Molecular Architecture and Functional Analysis of NetB, a Pore-forming Toxin from Clostridium perfringens. J Biol Chem 2013 Feb 1;288(5):3512-22.
[19] Coursodon CF, Glock RD, Moore KL, Cooper KK, Songer JG. TpeL-producing strains of Clostridium perfringens type A are highly virulent for broiler chicks. Anaerobe 2012 Feb;18(1):117-21.
[20] Saleh N, Fathalla SI, Nabil R, Mosaad AA. Clinicopathological and immunological studies on toxoids vaccine as a successful alternative in controlling clostridial infection in broilers. Anaerobe 2011;17(6):426-30.
[21] Lanckriet A, Timbermont L, Eeckhaut V, Haesebrouck F, Ducatelle R, Van Immerseel F. Variable protection after vaccination of broiler chickens against necrotic enteritis using supernatants of different Clostridium perfringens strains. Vaccine 2010;28(36):5920-3.
[22] Kulkarni RR, Parreira VR, Sharif S, Prescott JF. Immunization of broiler chickens against Clostridium perfringens-induced necrotic enteritis. Clin Vaccine Immunol 2007;14(9):1070-7.
[23] Jiang Y, Kulkarni RR, Parreira VR, Prescott JF. Immunization of broiler chickens against Clostridium perfringens-induced necrotic enteritis using purified recombinant immunogenic proteins. Avian Dis 2009;53(3):409-15.
[24] Jang SI, Lillehoj HS, Lee SH, Lee KW, Lillehoj EP, Hong YH, et al. Vaccination with Clostridium perfringens recombinant proteins in combination with Montanide ISA 71 VG adjuvant increases protection against experimental necrotic enteritis in commercial broiler chickens. Vaccine 2012;30(36):5401-6.
[25] Fernandes da Costa SP, Mot D, Bokori-Brown M, Savva CG, Basak AK, Van Immerseel F, et al. Protection against avian necrotic enteritis after immunisation with NetB genetic or formaldehyde toxoids. Vaccine 2013 Aug 20;31(37):4003-8.
[26] Williamson ED, Titball RW. A genetically engineered vaccine against the alpha-toxin of Clostridium perfringens protects mice against experimental gas-gangrene. Vaccine 1993 Sep;11(12):1253-8.
[27] Titball RW, Fearn AM, Williamson ED. Biochemical and immunological properties of the C-terminal domain of the alpha-toxin of Clostridium perfringens. FEMS Microbiol Lett 1993 Jun 1;110(1):45-50.
[28] Mot D, Timbermont L, Delezie E, Haesebrouck F, Ducatelle R, Van Immerseel F. Day-of-hatch vaccination is not protective against necrotic enteritis in broiler chickens. Avian Pathol 2013; 42:2, 179-184.
[29] Keyburn AL, Sheedy SA, Ford ME, Williamson MM, Awad MM, Rood JI, et al. Alpha-toxin of Clostridium perfringens is not an essential virulence factor in necrotic enteritis in chickens. Infect Immun 2006 Nov;74(11):6496-500.
[30] Keyburn AL, Portela RW, Sproat K, Ford ME, Bannam TL, Yan X, et al. Vaccination with recombinant NetB toxin partially protects broiler chickens from necrotic enteritis. Vet Res 2013;44(1):54.
[31] Jiang Y, Kulkarni RR, Parreira VR, Poppe C, Roland KL, Prescott JF. Assessment of 2 Salmonella enterica serovar Typhimurium-based vaccines against necrotic enteritis in reducing colonization of chickens by Salmonella serovars of different serogroups. Can J Vet Res 2010 Oct;74(4):264-70.
[32] Kulkarni RR, Parreira VR, Jiang YF, Prescott JF. A live oral recombinant Salmonella enterica serovar typhimurium vaccine expressing Clostridium perfringens antigens confers protection against necrotic enteritis in broiler chickens. Clin Vaccine Immunol 2010 Feb;17(2):205-14.
[33] Kulkarni RR, Parreira VR, Sharif S, Prescott JF. Oral immunization of broiler chickens against necrotic enteritis with an attenuated Salmonella vaccine vector expressing Clostridium perfringens antigens. Vaccine 2008 Aug 5;26(33):4194-203.
[34] Zekarias B, Mo H, Curtiss R, 3rd. Recombinant attenuated Salmonella enterica serovar typhimurium expressing the carboxy-terminal domain of alpha toxin from Clostridium perfringens induces protective responses against necrotic enteritis in chickens. Clin Vaccine Immunol 2008 May;15(5):805-16.

## Claims

1. A composition comprising:
a) a reduced toxicity NetB epitope polypeptide comprising amino acid sequence SEQ ID NO:2, or comprising a functional fragment of SEQ ID NO:2 having at least about 95% global sequence identity to SEQ ID NO:2 and comprising ETTQARGTNK (SEQ ID NO: 17); and
b) a reduced toxicity *Clostridium perfringens* alpha-toxin epitope polypeptide comprising the amino acid sequence SEQ ID NO:3, or comprising a functional fragment of SEQ ID NO:3 having at least about 95% global sequence identity to SEQ ID NO:3 and comprising the amino acid sequence of SEQ ID NO: 16.

2. The composition according to claim 1 wherein the NetB epitope polypeptide comprises at least one of the sequences:
a) YHAIXGNQLF (SEQ ID NO:4), wherein X is any amino acid other than Y;
b) FMKSXLYNNG (SEQ ID NO:6), wherein X is any amino acid other than R; or
c) YILNXETTQW (SEQ ID NO:8), wherein X is any amino acid other than W; optionally wherein any X is A.

3. The composition according to any preceding claim wherein the alpha-toxin epitope polypeptide comprises a sequence of at least 5 contiguous amino acids from SEQ ID NO:18.

4. The composition according to any preceding claim wherein the alpha-toxin epitope polypeptide comprises the amino acid sequence SEQ ID NO:18, or a functional fragment or variant thereof having at least 34% global sequence identity to SEQ ID NO:18.

5. The composition according to any preceding claim wherein at least one polypeptide is a recombinant polypeptide, optionally wherein the alpha-toxin epitope polypeptide is expressed as a fusion protein with GST or a His-tag and/or wherein the NetB epitope polypeptide is expressed with a His-tag or as a fusion protein with GST, and/or comprising a fusion protein which comprises the reduced toxicity NetB epitope polypeptide and the reduced toxicity *C*. *perfringens* alpha-toxin epitope polypeptide.

6. A polynucleotide encoding:
a) a reduced toxicity NetB epitope polypeptide comprising amino acid sequence SEQ ID NO:2, or comprising a functional fragment of SEQ ID NO:2 having at least about 95% global sequence identity to SEQ ID NO:2 and comprising ETTQARGTNK (SEQ ID NO: 17); and
b) a reduced toxicity *C*. *perfringens* alpha-toxin epitope polypeptide comprising the amino acid sequence SEQ ID NO:3, or comprising a functional fragment of SEQ ID NO:3 having at least about 95% global sequence identity to SEQ ID NO:3 and comprising the amino acid sequence of SEQ ID NO: 16.

7. A vector comprising a polynucleotide according to claim 6.

8. A cell comprising a composition according to any of claims 1-5 and/or a polynucleotide according to claim 6 and/or a vector according to claim 7, optionally which is a *Salmonella, Bacillus, Lactococcus or Lactobacillus* cell.

9. A vaccine composition comprising a composition according to any of claims 1-5 and/or a polynucleotide according to claim 6 and/or a vector according to claim 7 and/or a cell according to claim 8, optionally which comprises a subunit vaccine, further optionally which is an animal feed.

10. The composition according to any of claims 1-5 or 9, or a polynucleotide according to claim 6, or a vector according to claim 7, or a cell according to claim 8, for use in (i) therapy, (ii) eliciting an immune response against *C*. *perfringens,* and/or (iii) a method of vaccinating a subject against infection by *C*. *perfringens,* optionally wherein the subject is poultry, for example of the genus *Gallus*, for example of the species *Gallus gallus.*

11. A reduced toxicity NetB epitope polypeptide in combination with a reduced toxicity *C*. *perfringens* alpha-toxin epitope polypeptide for use in a method of (i) eliciting an immune response against *C*. *perfringens*, and/or (ii) vaccinating a subject against infection by *C*. *perfringens*, wherein the reduced toxicity NetB epitope polypeptide comprises amino acid sequence SEQ ID NO:2, or comprises a functional fragment of SEQ ID NO:2 having at least about 95% global sequence identity to SEQ ID NO:2 and comprising ETTQARGTNK (SEQ ID NO: 17); and wherein the reduced toxicity *C*. *perfringens* alpha-toxin epitope polypeptide comprises the amino acid sequence SEQ ID NO:3, or comprises a functional fragment of SEQ ID NO:3 having at least about 95% global sequence identity to SEQ ID NO:3 and comprises the amino acid sequence of SEQ ID NO: 16.

12. The reduced toxicity NetB epitope polypeptide in combination with a reduced toxicity *C. perfringens* alpha-toxin epitope polypeptide according to claim 1 for use in a method according to claim 11, wherein the polypeptides are administered sequentially to the subj ect.

13. A kit comprising:
a) a composition according to any of claims 1-5 or 9 or 10, or a polynucleotide according to claim 6, or a vector according to claim 7, or a cell according to claim 8; or
b) a reduced toxicity NetB epitope polypeptide and a reduced toxicity *C*. *perfringens* alpha-toxin epitope polypeptide, wherein the reduced toxicity NetB epitope polypeptide comprises amino acid sequence SEQ ID NO:2, or comprises a functional fragment of SEQ ID NO:2 having at least about 95% global sequence identity to SEQ ID NO:2 and comprises ETTQARGTNK (SEQ ID NO: 17); and wherein the reduced toxicity *C. perfringens* alpha-toxin epitope polypeptide comprises the amino acid sequence SEQ ID NO:3, or comprises a functional fragment of SEQ ID NO:3 having at least about 95% global sequence identity to SEQ ID NO:3 and comprises SEQ ID NO: 16.

14. A composition according to any of claims 1-5 or 9 or 10, or a reduced toxicity NetB epitope polypeptide in combination with a reduced toxicity *C. perfringens* alpha-toxin epitope polypeptide for use in a method of producing in a subject in need thereof antibodies capable of binding to SEQ ID NO:1 and antibodies capable of binding to SEQ ID NO:18 and/or 3, wherein the reduced toxicity NetB epitope polypeptide comprises amino acid sequence SEQ ID NO:2, or comprises a functional fragment of SEQ ID NO:2 having at least about 95% global sequence identity to SEQ ID NO:2 and comprises ETTQARGTNK (SEQ ID NO: 17); and wherein the reduced toxicity *C*. *perfringens* alpha-toxin epitope polypeptide comprises the amino acid sequence SEQ ID NO:3, or comprises a functional fragment of SEQ ID NO:3 having at least about 95%global sequence identity to SEQ ID NO:3 and comprising the amino acid sequence SEQ ID NO: 16.

## Patentansprüche

1. Zusammensetzung, umfassend:
a) ein NetB-Epitop-Polypeptid mit reduzierter Toxizität, umfassend Aminosäuresequenz SEQ ID NO: 2 oder umfassend ein funktionelles Fragment von SEQ ID NO: 2 mit mindestens etwa 95 % globaler Sequenzidentität zu SEQ ID NO: 2 und umfassend ETTQARGTNK (SEQ ID NO: 17), und
b) ein *Clostridium* perfringens-alpha-Toxin-Epitop-Polypeptid mit verringerter Toxizität, umfassend die Aminosäuresequenz SEQ ID NO: 3 oder umfassend ein funktionelles Fragment von SEQ ID NO: 3 mit mindestens etwa 95 % globaler Sequenzidentität zu SEQ ID NO: 3 und umfassend die Aminosäuresequenz von SEQ ID NO: 16.

2. Zusammensetzung nach Anspruch 1, wobei das NetB-Epitop-Polypeptid mindestens eine der Sequenzen umfasst:
a) YHAIXGNQLF (SEQ ID NO: 4), wobei X eine beliebige Aminosäure außer Y ist,
b) FMKSXLYNNG (SEQ ID NO: 6), wobei X eine beliebige Aminosäure außer R ist, oder
c) YILNXETTQW (SEQ ID NO: 8), wobei X eine beliebige Aminosäure außer W ist, wobei optional jedes X A ist.

3. Zusammensetzung nach einem vorhergehenden Anspruch, wobei das alpha-Toxin-EpitopPolypeptid eine Sequenz von mindestens 5 zusammenhängenden Aminosäuren aus SEQ ID NO: 18 umfasst.

4. Zusammensetzung nach einem vorhergehenden Anspruch, wobei das alpha-Toxin-EpitopPolypeptid die Aminosäuresequenz SEQ ID NO: 18 oder ein funktionelles Fragment oder eine Variante davon mit mindestens 34 % globaler Sequenzidentität zu SEQ ID NO: 18 umfasst.

5. Zusammensetzung nach einem vorhergehenden Anspruch, worin mindestens ein Polypeptid ein rekombinantes Polypeptid ist, wobei optional das alpha-Toxin-Epitop-Polypeptid als Fusionsprotein mit GST oder einem His-Tag exprimiertwird, und/oder wobei das NetB-Epitop Polypeptid mit einem His-Tag oder als Fusionsprotein mit GST exprimiert wird und/oder ein Fusionsprotein umfassend, das das NetB-Epitop-Polypeptid mit reduzierter Toxizität und das *C*. *perfringens*-alpha-Toxin-Epitop-Polypeptid mit reduzierter Toxizität umfasst.

6. Polynukleotid, codierend:
a) ein NetB-Epitop-Polypeptid mit reduzierter Toxizität, umfassend Aminosäuresequenz SEQ ID NO: 2 oder umfassend ein funktionelles Fragment von SEQ ID NO: 2 mit mindestens etwa 95 % globaler Sequenzidentität zu SEQ ID NO: 2 und umfassend ETTQARGTNK (SEQ ID NO: 17), und
b) ein *C*. *perfringens*-alpha-Toxin-Epitop-Polypeptid mit reduzierter Toxizität, umfassend die Aminosäuresequenz SEQ ID NO: 3 oder umfassend ein funktionelles Fragment von SEQ ID NO: 3 mit mindestens etwa 95 % globaler Sequenzidentität zu SEQ ID NO: 3 und umfassend die Aminosäuresequenz von SEQ ID NO: 16.

7. Vektor, umfassend ein Polynukleotid nach Anspruch 6.

8. Zelle, umfassend eine Zusammensetzung nach einem der Ansprüche 1 bis 5 und/oder ein Polynukleotid nach Anspruch 6 und/oder einen Vektor nach Anspruch 7, welche optional eine *Salmonella-, Bacillus-, Lactococcus-* oder *Lactobacillus*-Zelle ist.

9. Impfstoffzusammensetzung, umfassend eine Zusammensetzung nach einem der Ansprüche 1 bis 5 und/oder ein Polynukleotid nach Anspruch 6 und/oder einen Vektor nach Anspruch 7 und/oder eine Zelle nach Anspruch 8, die optional einen Untereinheitenimpfstoff umfasst, welche weiter optional ein Tierfutter ist.

10. Zusammensetzung nach einem der Ansprüche 1 bis 5 oder 9 oder Polynukleotid nach Anspruch 6 oder Vektor nach Anspruch 7 oder Zelle nach Anspruch 8 zur Verwendung (i) in der Therapie, (ii) beim Auslösen einer Immunantwort gegen *C*. *perfringens* und/oder (iii) in einem Verfahren der Impfung eines Subjekts gegen eine Infektion mit *C*. *perfringens,* wobei optional das Subjekt Geflügel ist, zum Beispiel der Gattung Gallus, zum Beispiel der Art Gallus gallus.

11. NetB-Epitop-Polypeptid mit reduzierter Toxizität in Kombination mit einem *C*. *perfringens-*alpha-Toxin-Epitop-Polypeptid mit reduzierter Toxizität zur Verwendung in einem Verfahren (i) zum Auslösen einer Immunantwort gegen *C*. *perfringens* und/oder (ii) der Impfung eines Subjekts gegen eine Infektion mit *C*. *perfringens,* wobei das NetB-Epitop-Polypeptid mit reduzierter Toxizität die Aminosäuresequenz SEQ ID NO: 2 umfasst oder ein funktionelles Fragment von SEQ ID NO: 2 umfasst mit mindestens etwa 95 % globaler Sequenzidentität zu SEQ ID NO: 2 und umfassend ETTQARGTNK (SEQ ID NO: 17), und wobei das *C*. *perfringens*-alpha-Toxin-Epitop-Polypeptid mit reduzierter Toxizität die Aminosäuresequenz SEQ ID NO: 3 umfasst oder ein funktionelles Fragment von SEQ ID NO: 3 mit mindestens etwa 95 % globaler Sequenzidentität zu SEQ ID NO: 3 und die Aminosäuresequenz von SEQ ID NO: 16 umfasst.

12. NetB-Epitop-Polypeptid mit reduzierter Toxizität in Kombination einem *C*. *perfringens-*alpha-Toxin-Epitop-Polypeptid mit reduzierter Toxizität nach Anspruch 1 zur Verwendung in einem Verfahren nach Anspruch 11, wobei die Polypeptide dem Subjekt nacheinander verabreicht werden.

13. Kit, umfassend:
a) eine Zusammensetzung nach einem der Ansprüche 1 bis 5 oder 9 oder 10 oder ein Polynukleotid nach Anspruch 6 oder einen Vektor nach Anspruch 7 oder eine Zelle nach Anspruch 8 oder
b) ein NetB-Epitop-Polypeptid mit reduzierter Toxizität und ein *C*. *perfringens-*alpha-Toxin-Epitop-Polypeptid mit reduzierter Toxizität, wobei das NetB-Epitop-Polypeptid mit reduzierter Toxizität die Aminosäuresequenz SEQ ID NO: 2 umfasst oder ein funktionelles Fragment von SEQ ID NO: 2 mit mindestens etwa 95 % globaler Sequenzidentität zu SEQ ID NO: 2 umfasst und ETTQARGTNK (SEQ ID NO: 17) umfasst, und wobei das *C*. *perfringens*-alpha-Toxin-Epitop-Polypeptid die Aminosäuresequenz SEQ ID NO: 3 oder ein funktionelles Fragment von SEQ ID NO: 3 mit mindestens etwa 95 % globaler Sequenzidentität zu SEQ ID NO: 3 umfasst und SEQ ID NO: 16 umfasst.

14. Zusammensetzung nach einem der Ansprüche 1 bis 5 oder 9 oder 10 oder NetB-Epitop-Polypeptid mit reduzierter Toxizität in Kombination mit *C*. *perfringens*-alpha-Toxin-Epitop-Polypeptid mit reduzierter Toxizität zur Verwendung in einem Verfahren zur Erzeugung von Antikörpern in einem diese benötigenden Subjekt, wobei die Antikörper in der Lage sind, an SEQ ID NO: 1 zu binden, und von Antikörpern, die in der Lage sind, an SEQ ID NO: 18 und/oder 3 zu binden, wobei das NetB-Epitop-Polypeptid mit reduzierter Toxizität Aminosäuresequenz SEQ ID NO: 2 umfasst oder ein funktionelles Fragment von SEQ ID NO: 2 mit mindestens etwa 95 % globaler Sequenzidentität zu SEQ ID NO: 2 umfasst und ETTQARGTNK (SEQ ID NO: 17) umfasst, und wobei das *C*. *perfringens*-alpha-Toxin-Epitop-Polypeptid mit reduzierter Toxizität die Aminosäuresequenz SEQ ID NO: 3 umfasst oder ein funktionelles Fragment von SEQ ID NO: 3 mit mindestens etwa 95 % globaler Sequenzidentität zu SEQ ID NO: 3 umfasst und umfassend die Aminosäuresequenz SEQ ID NO: 16.

## Revendications

1. Composition comprenant :
a) un polypeptide épitopique NetB à toxicité réduite comprenant la séquence d'acides aminés SEQ ID No. 2, ou comprenant un fragment fonctionnel de SEQ ID No. 2 ayant au moins environ 95 % d'identité de séquence globale à la SEQ ID No. 2 et comprenant ETTQARGTNK (SEQ ID No. 17) ; et
b) un polypeptide épitopique de toxine alpha de *Clostridium perfringens* à toxicité réduite comprenant la séquence d'acides aminés SEQ ID No.3, ou comprenant un fragment fonctionnel de SEQ ID No.3 ayant au moins environ 95 % d'identité de séquence globale à la SEQ ID No.3 et comprenant la séquence d'acides aminés de SEQ ID No. 16.

2. Composition selon la revendication 1, dans laquelle le polypeptide épitopique NetB comprend au moins l'une des séquences :
a) YHAIXGNQLF (SEQ ID No. 4), dans laquelle X est un acide aminé différent de Y ;
b) FMKSXLYNNG (SEQ ID No. 6), dans laquelle X est un acide aminé différent de R ; ou
c) YILNXETTQW (SEQ ID No. 8), dans laquelle X est un acide aminé différent de W ; dans laquelle éventuellement tout X est A.

3. Composition selon l'une quelconque des revendications précédentes, dans laquelle le polypeptide épitopique de toxine alpha comprend une séquence d'au moins 5 acides aminés contigus provenant de la SEQ ID No. 18.

4. Composition selon l'une quelconque des revendications précédentes, dans laquelle le polypeptide épitopique de toxine alpha comprend la séquence d'acides aminés SEQ ID No. 18, ou un fragment fonctionnel ou une variante de celui-ci ayant au moins 34 % d'identité de séquence globale à la SEQ ID No. 18.

5. Composition selon l'une quelconque des revendications précédentes, dans laquelle au moins un polypeptide est un polypeptide recombinant, dans laquelle le polypeptide épitopique de toxine alpha est éventuellement exprimé en tant que protéine de fusion avec GST ou une étiquette His et/ou dans laquelle le polypeptide épitopique NetB est exprimé avec une étiquette His ou en tant que protéine de fusion avec GST, et/ou comprenant une protéine de fusion qui comprend le polypeptide épitopique NetB à toxicité réduite et le polypeptide épitopique de toxine alpha de *C*. *perfringens* à toxicité réduite.

6. Polynucléotide codant pour :
a) un polypeptide épitopique NetB à toxicité réduite comprenant la séquence d'acides aminés SEQ ID No. 2, ou comprenant un fragment fonctionnel de SEQ ID No. 2 ayant au moins environ 95 % d'identité de séquence globale à la SEQ ID No. 2 et comprenant ETTQARGTNK (SEQ ID No. 17) ; et
b) un polypeptide épitopique de toxine alpha de *C. perfringens* à toxicité réduite comprenant la séquence d'acides aminés SEQ ID No. 3, ou comprenant un fragment fonctionnel de SEQ ID No .3 ayant au moins environ 95 % d'identité de séquence globale à la SEQ ID No. 3 et comprenant la séquence d'acides aminés de SEQ ID No. 16.

7. Vecteur comprenant un polynucléotide selon la revendication 6.

8. Cellule comprenant composition selon l'une quelconque des revendications 1 à 5 et/ou polynucléotide selon la revendication 6 et/ou vecteur selon la revendication 7, qui est éventuellement une cellule de type *Salmonella, Bacillus, Lactococcus ou Lactobacillus.*

9. Composition de vaccin comprenant la composition selon l'une quelconque des revendications 1 à 5 et/ou un polynucléotide selon la revendication 6 et/ou un vecteur selon la revendication 7 et/ou une cellule selon la revendication 8, qui comprend en outre un vaccin sous-unitaire, qui est en outre éventuellement un aliment pour animaux.

10. Composition selon l'une quelconque des revendications 1 à 5 ou 9, ou polynucléotide selon la revendication 6, ou vecteur selon la revendication 7, ou cellule selon la revendication 8, destiné à être utilisé (i) dans un traitement, (ii) dans le déclenchement d'une réponse immunitaire contre C. ***perfringens,*** et/ou (iii) dans un procédé de vaccination d'un sujet contre une infection par C. ***perfringens,*** le sujet étant éventuellement une volaille, par exemple du genre *Gallus*, par exemple de l'espèce *Gallus gallus.*

11. Polypeptide épitopique NetB à toxicité réduite en combinaison avec un polypeptide épitopique de toxine alpha de *C. perfringens* à toxicité réduite destiné à être utilisé dans un procédé de (i) déclenchement d'une réponse immunitaire contre C. *perfringens,* et/ou (ii) vaccination d'un sujet contre une infection par C. *perfringens,* dans lequel le polypeptide épitopique NetB à toxicité réduite comprend la séquence d'acides aminés SEQ ID No. 2, ou comprend un fragment fonctionnel de SEQ ID No. 2 ayant au moins environ 95 % d'identité de séquence globale à la SEQ ID No. 2 et comprenant ETTQARGTNK (SEQ ID No. 17) ; et dans lequel le polypeptide épitopique de toxine alpha de *C. perfringens* à toxicité réduite comprend la séquence d'acides aminés SEQ ID No. 3, ou comprend un fragment fonctionnel de SEQ ID No. 3 ayant au moins environ 95 % d'identité de séquence globale à la SEQ ID No. 3 et comprend la séquence d'acides aminés de SEQ ID No. 16.

12. Polypeptide épitopique NetB à toxicité réduite en combinaison un polypeptide épitopique de toxine alpha de *C. perfringens* à toxicité réduite selon la revendication 1 destiné à être utilisé dans un procédé selon la revendication 11, dans lequel les polypeptides sont administrés de manière séquentielle au sujet.

13. Kit comprenant :
a) la composition selon l'une quelconque des revendications 1 à 5 ou 9 ou 10, ou un polynucléotide selon la revendication 6, ou un vecteur selon la revendication 7, ou une cellule selon la revendication 8 ; ou
b) un polypeptide épitopique NetB à toxicité réduite et un polypeptide épitopique de toxine alpha de *C. perfringens* à toxicité réduite, dans lequel le polypeptide épitopique NetB à toxicité réduite comprend la séquence d'acides aminés SEQ ID No. 2, ou comprend un fragment fonctionnel de SEQ ID No. 2 ayant au moins environ 95 % d'identité de séquence globale à la SEQ ID No. 2 et comprend ETTQARGTNK (SEQ ID No. 17) ; et dans lequel le polypeptide épitopique de toxine alpha de *C. perfringens* à toxicité réduite comprend la séquence d'acides aminés SEQ ID No. 3, ou comprend un fragment fonctionnel de SEQ ID No. 3 ayant au moins environ 95 % d'identité de séquence globale à la SEQ ID No. 3 et comprend la SEQ ID No. 16.

14. Composition selon l'une quelconque des revendications 1 à 5 ou 9 ou 10, ou polypeptide épitopique NetB à toxicité réduite en combinaison avec un polypeptide épitopique de toxine alpha de *C. perfringens* à toxicité réduite destiné à être utilisé dans un procédé de production chez un sujet en ayant besoin d'anticorps capables de se lier à la SEQ ID No. 1 et d'anticorps capables de se lier à la SEQ ID No. 18 et/ou 3, le polypeptide épitopique NetB à toxicité réduite comprenant la séquence d'acides aminés SEQ ID No. 2, ou comprenant un fragment fonctionnel de SEQ ID No. 2 ayant au moins environ 95 % d'identité de séquence globale à la SEQ ID No. 2 et comprenant ETTQARGTNK (SEQ ID No. 17) ; et le polypeptide épitopique de toxine alpha de *C. perfringens* à toxicité réduite comprenant la séquence d'acides aminés SEQ ID No. 3, ou comprenant un fragment fonctionnel de SEQ ID No. 3 ayant au moins environ 95 % d'identité de séquence globale à la SEQ ID No. 3 et comprenant la séquence d'acides aminés SEQ ID No. 16.
